Europäisches Patentamt

European Patent Office

Office européen des brevets

(11) Veröffentlichungsnummer: **0 255 926**

**A2**

(12) **EUROPÄISCHE PATENTANMELDUNG**

(21) Anmeldenummer: 87111159.7

(22) Anmeldetag: 01.08.87

(51) Int. Cl.⁴: **A61L 31/00 , A61L 15/03**

Patentansprüche für folgenden Vertragsstaaten:
AT + ES + GR.

(30) Priorität: 07.08.86 DE 8621153 U
18.10.86 DE 3635453

(43) Veröffentlichungstag der Anmeldung:
17.02.88 Patentblatt 88/07

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Seibert, Gerhard, Prof. Dr.
Gläserweg 21
D-6100 Darmstadt(DE)
Erfinder: Schrinner, Elmar, Dr.
Hedwigstrasse 2
D-6200 Wiesbaden(DE)

(54) **Antibakterielle Klebefolie und Verfahren zu ihrer Herstellung.**

(57) Eine Klebefolie, die mit einem antibakteriell wirksamen Chinoloncarbonsäurederivat imprägniert ist, sowie Verfahren zu ihrer Herstellung werden beschrieben.

FIG. 2

µg Ofloxacin / ml Elutionsflüssigkeit

Stunden

## Antibakterielle Klebefolie und Verfahren zu ihrer Herstellung

Die Erfindung betrifft eine Klebefolie, die mit einem Chinoloncarbonsäurederivat antibakteriell ausgerüstet ist. Sie kann sowohl als Operationsinzisionsfolie als auch als postoperativer Wundverband eingesetzt werden.

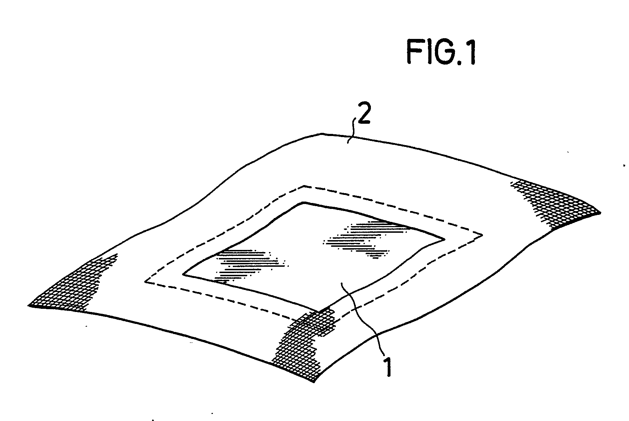

Es ist bekannt, daß heute üblicherweise während der Operation der Patient mit einem Operationstuch bedeckt wird. Derartige Operationstücher, an deren Luftdurchlässigkeit und Feuchtigkeitsaufnahmevermögen hohe Anforderungen gestellt werden, sind beispielsweise aus der Britischen Patentanmeldung 20 80 197 bekannt. Fig. 1 entspricht dem Stand der Technik und zeigt, daß das Operationstuch (2) an der zur Operation vorgesehenen Stelle eine Öffnung enthält, in die eine Operationsinzisionsfolie (1) eingesetzt ist. Diese Folie, die bei der Operation durchschnitten wird, kann aus einem durchsichtigen Folienmaterial, z.B. einer Hochdruckpolyethylenfolie, einer Polyesterfolie, aber auch aus einem beliebigen anderen Folienmaterial bestehen, z.B. auch Polyurethan. Die dem Körper des Patienten zugewandte Seite der Operationsinzisionsfolie ist mit einem Klebstoff versehen, der nach dem Andrücken eine sichere Haftung am Körper des Patienten gewährleistet. Durch derartige Klebefolien wird eine dichte Barriere zwischen der Operationswunde und der umgebenden Haut errichtet. Transparente Klebefolien finden außerdem auch als postoperativer Wundverband in der Abdominalchirurgie, in der plastischen Chirurgie, in der Unfallchirurgie und zur Versorgung von Verbrennungswunden Anwendung. Insbesondere Polyurethanfolien zeigen dabei Vorteile gegenüber einem konventionellen Verband, weil sie wasser-und bakteriendicht, gas-und wasserdampfdurchlässig sowie elastisch sind (vgl. A. Schröder et al., medwelt 1986, 37, 500 - 552). Allerdings werden die auf der Haut befindlichen Bakterien durch diese Klebefolie lediglich abgedeckt und können deshalb eine Wundinfektion verursachen.

Gegenstand der Erfindung ist deshalb eine Klebefolie, die mit einem antibakteriell wirksamen Chinoloncarbonsäurederivat imprägniert ist, sowie ein Verfahren zu ihrer Herstellung, das dadurch gekennzeichnet ist, daß man die Klebefolie in die wäßrige Lösung des Chinoloncarbonsäurederivates eintaucht oder daß man das Chinoloncarbonsäurederivat in den Kleber einarbeitet und auf die Folie aufträgt.

Geeignete Chinoloncarbonsäurederivate sind in dem Europäischen Patent 47 005 (entsprechend US-Patent Nr. 4 382 892) beschrieben. Besonders geeignete Chinoloncarbonsäurederivate sind Ofloxacin, Norfloxacin, Ciprofloxacin und Pefloxacin.

Sie sind in der Klebefolie selbst und/oder im Klebstoff enthalten. Im allgemeinen wird die Menge des antibakteriellen Chinoloncarbonsäurederivates eine Konzentration von 1 mg/cm$^2$ nicht übersteigen.

Für die Imprägnierung eignen sich allgemein übliche Klebefolien. Bevorzugt wird eine Polyurethanklebefolie. Die Imprägnierung der Klebefolie mit dem Chinoloncarbonsäurederivat ist äußerst einfach. Es reicht aus, die Folie mehrere Stunden in eine wäßrige Lösung des Chinoloncarbonsäurederivats einzuhängen. Im allgemeinen wählt man dazu Lösungen, die das Chinoloncarbonsäurederivat in einer Konzentration zwischen 0,1 und 10 % enthalten. Die Verweildauer der Folie in der Lösung sollte zweckmäßigerweise 10 bis 20 Stunden betragen, jedoch sind auch kürzere oder längere Zeiträume möglich. Danach wird die Klebefolie abgespült und kann dann als Operationsinzisionsfolie oder als postoperativer Wundverband eingesetzt werden. Das Auftragen des Klebers, welcher das Chinoloncarbonsäurederivat enthält, auf eine Folie erfolgt nach an sich bekannten Methoden.

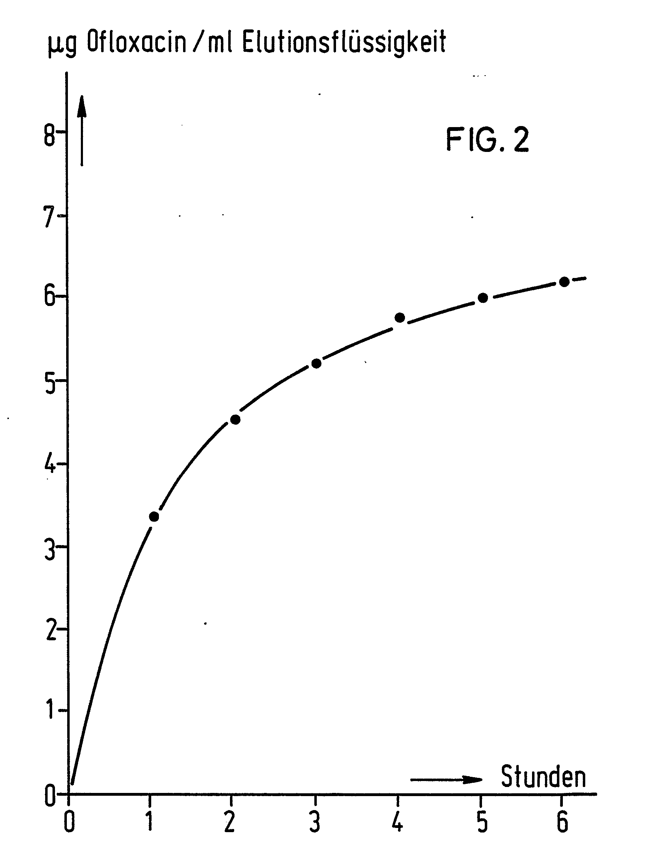

Für die erfindungsgemäße Klebefolie ist es kennzeichnend, daß sie den aufgenommenen Wirkstoff nur langsam wieder freigibt und dadurch über einen langen Zeitraum die antibakterielle Wirksamkeit behält. Fig. 2 zeigt die Freigabe von Ofloxacin aus einer Polyurethanklebefolie, die mehrere Stunden mit destilliertem Wasser eluiert wird. Auch nach einer 6-stündigen Behandlung enthält die Folie noch so viel Wirkstoff, daß nach Auflegen eines Stückes der Folie auf eine mit einem bakteriellen Krankheitserreger beimpfte Nährbodenfläche ein deutlicher Wachstumshemmhof entstand.

Es ist zwar bereits schon vorgeschlagen worden, Kunststoffimplantate für medizinische Zwecke mit einem Gyrasehemmer auszustatten. Beispielsweise ist darauf hingewiesen worden, daß man Herzschrittmacher oder Venenkatheter mit einem Lack überziehen kann, der einen Gyrasehemmer enthält. Damit ist dieses Implantat dann antibakteriell ausgestattet (Stille, Münch. med. Wsch. 128 (1986), Beilage 24, Seite 7).

Demgegenüber ist es überraschend, daß es zur antibakteriellen Ausstattung einer Klebefolie der Anwendung eines speziellen Lackes überhaupt nicht bedarf. Der Ersatz des bisher vorgeschlagenen gyrasehemmerhaltigen Überzugslackes durch ein einfaches Imprägnierungsverfahren ist eine wichtige Voraussetzung für die preiswerte Herstellung der erfindungsgemäßen Klebefolie. Sie ist ein ausgezeichneter Schutz gegen Wundinfektionen bei chirurgischen Eingriffen.

Beispiel

Ein Stück einer Polyurethanklebefolie ®Opsite (Smith & Nephew Ltd., Hull, England) von der Größe 15 x 27 cm wird über 18 Stunden in 500 ml einer 1 %igen wäßrigen Lösung (pH = 7) von Ofloxacin gelegt. Anschließend wird die Folie mit 3 x 800 ml destilliertem Wasser gespült und dann in 800 ml Wasser aufgenommen. Das jetzt aus der Folie eluierte Ofloxacin wird fluorimetrisch bestimmt. In Fig. 2 ist die Menge des pro ml Elutionsflüssigkeit abgegebenen Ofloxacins in Abhängigkeit von der Zeit dargestellt. Daraus ist zu ersehen, daß Ofloxacin über mindestens 6 Stunden kontinuierlich freigesetzt wird. Es verbleibt auch nach dieser Zeit noch soviel Ofloxacin in der Folie, daß nach dem Auflegen eines 1 cm² großen Stückes der Folie auf einen Nährboden, der als Testkeim den Krankheitserreger Klebsiella aerogenes 1082 E enthält im Bereich der Folienauflage sowie in der unmittelbaren Umgebung eine Hemmung des Bakterienwachstums zu beobachten ist.

## Ansprüche

1. Klebefolie, dadurch gekennzeichnet, daß sie mit einem antibakteriell wirksamen Chinoloncarbonsäurederivat imprägniert ist.

2. Klebefolie nach Anspruch 1, dadurch gekennzeichnet, daß sie als Chinoloncarbonsäurederivat Ofloxacin, Norfloxacin, Ciprofloxacin oder Pefloxacin enthält.

3. Klebefolie nach Anspruch 1, dadurch gekennzeichnet, daß das antibakterille Chinoloncarbonsäurederivat in der Folie und/oder im Klebstoff enthalten ist.

4. Klebefolie nach Anspruch 1, dadurch gekennzeichnet, daß sie das antibakterielle Chinoloncarbonsäurederivat in einer Menge bis 1 mg/cm² enthält.

5. Klebefolie nach Anspruch 1, dadurch gekennzeichnet, daß sie in ein Operationstuch als Operationsinzisionsfolie eingesetzt ist.

6. Klebefolie nach Anspruch 1, dadurch gekennzeichnet, daß sie als postoperativer Wundverband eingesetzt wird.

7. Verfahren zur Herstellung einer Klebefolie gemäß Anspruch 1, dadurch gekennzeichnet, daß man die Klebefolie in die wäßrige Lösung des Chinoloncarbonsäurederivates eintaucht oder daß man das Chinoloncarbonsäurederivat in den Kleber einarbeitet und auf die Folie auftragt.

Patentansprüche für die folgenden Vertragsstaaten: Griechenland, Spanien und Österreich:

1. Verfahren zur Herstellung einer Klebefolie, dadurch gekennzeichnet, daß man eine Klebefolie in die wäßrige Lösung eines antibakteriell wirksamen Chinoloncarbonsäurederivats eintaucht oder daß man das Chinoloncarbonsäurederivat in den Kleber einarbeitet und auf die Folie aufträgt.

2. Verfahren gemäß Anspruch 1, dadurch gekennzeichnet, daß als Chinoloncarbonsäurederivat Ofloxacin, Norfloxacin, Ciprofloxacin oder Pefloxacin eingesetzt wird.

3. Verfahren gemäß Anspruch 1, dadurch gekennzeichnet, daß man eine Klebefolie, die das Chinoloncarbonsäurederivat in einer Menge bis 1 mg/cm² enthält, herstellt.

# FIG.1

μg Ofloxacin / ml Elutionsflüssigkeit

FIG. 2

Stunden